19 Europäisches Patentamt

European Patent Office

Office européen des brevets

11 Veröffentlichungsnummer: **0 248 238 B1**

12 **EUROPÄISCHE PATENTSCHRIFT**

45 Veröffentlichungstag der Patentschrift: **11.11.92**

21 Anmeldenummer: **87106872.2**

22 Anmeldetag: **12.05.87**

51 Int. Cl.5: **C12P 13/06**, C12N 1/20,
//(C12N1/20,C12R1:15)

54 Verfahren zur fermentativen Herstellung von L-Isoleucin aus D,L-alpha-Hydroxybutyrat.

30 Priorität: **06.06.86 DE 3619111**

43 Veröffentlichungstag der Anmeldung:
**09.12.87 Patentblatt 87/50**

45 Bekanntmachung des Hinweises auf die
Patenterteilung:
**11.11.92 Patentblatt 92/46**

84 Benannte Vertragsstaaten:
**BE CH DE FR GB LI NL SE**

56 Entgegenhaltungen:
DE-A- 1 543 877
DE-A- 1 910 427
GB-A- 2 084 998

PATENT ABSTRACTS OF JAPAN, Band 8, Nr.
222 (C-246)[1659], 9. Oktober 1984; & JP-A-59
106 294 (KYOWA HAKKO KOGYO K.K.)
19-06-1984

PATENT ABSTRACTS OF JAPAN, Band 8, Nr.
222 (C-246)[1659], 9. Oktober 1984; & JP-A-59
106 295 (KYOWA HAKKO KOGYO K.K.)
19-06-1984

73 Patentinhaber: **Degussa Aktiengesellschaft
Weissfrauenstrasse 9
W-6000 Frankturt am Main 1(DE)**

84 Benannte Vertragsstaaten:
**BE CH DE FR GB LI NL SE**

73 Patentinhaber: **Forschungszentrum Jülich
GmbH
Postfach 1913
W-5170 Jülich(DE)**

84 Benannte Vertragsstaaten:
**BE CH DE FR GB LI NL SE**

72 Erfinder: **Scheer, Elisabeth
Koenigshuegel 25
W-5100 Aachen(DE)**
Erfinder: **Sahm, Hermann, Prof.
Wendelinusstrasse 71
W-5170 Jülich(DE)**
Erfinder: **Eggeling, Lothar, Dr.
Schneppruthweg 7
W-5170 Jülich(DE)**

Erfinder: **Kircher, Manfred, Dr.**
**Goldschmiedeweg 6**
**W-4800 Bielefeld 12(DE)**
Erfinder: **Leuchtenberger, Wolfgang, Dr.**
**Geschwister-Scholl-Strasse 1**
**W-6454 Bruckköbel(DE)**

**Beschreibung**

Die Erfindung betrifft ein Verfahren zur fermentativen Herstellung von L-Isoleucin aus dem racemischen Gemisch von D- und L-α-Hydroxybutyrat durch D-Lactat verwertende Mutanten der Gattung Corynebacterium oder Brevibacterium.

Die Aminosäure L-Isoleucin ist für Mensch und Tier essentiell und findet als Komponente verschiedener Nährgemische medizinischer Zweckbestimmung breite Verwendung. Ferner wird L-Isoleucin als Zugabe zu menschlicher Nahrung und als Tierfutter sowie als Reagens für die pharmazeutische und chemische Industrie benutzt.

Es ist bekannt, daß sich L-Isoleucin fermentativ aus verschiedenen Kohlenhydraten wie Glucose, Fructose, Stärke-Hydrolysat, Cellulose-Hydrolysat und Melassen oder Kohlenwasserstoffen wie n-Paraffinenfermentativ aus verschiedenen Vorstufen der L-Isoleucinbiosynthese herstellen läßt. Dabei finden hauptsächlich Vertreter der Gattung Corynebacterium, Brevibacterium oder Arthrobacter Verwendung, die sich in einer Nährlösung befinden, die eine Vorstufe, sowie eine Kohlenstoff- und Energiequelle, eine Stickstoffquelle, Vitamine und andere Komponenten enthält, und die die Vorstufen teilweise zu L-Isoleucin umwandeln und in der Nährlösung anhäufen. Als Vorstufen finden Threonin, α-Aminobutyrat, α-Hydroxybutyrat oder α-Ketobutyrat Verwendung. Die genannten Vorstufen (mit Ausnahme von α-Ketobutyrat) können jeweils in der L- und D-Form vorliegen, sowie als Racemat. Technisch leicht zugänglich sind die Vorstufen in Form ihres Racemats, so daß für den effektiven Einsatz von Vorstufen die vollständige Nutzung der D- und L-Form gegeben sein muß.

Mit Brevibacterium thiogenitalis konnte bei Einsatz von 30 g/l Hydroxybutyrat als Vorstufe eine maximale L-Isoleucin-Konzentration von 15.8 g/l erzielt werden (US-PS 4 329,427).

Dieses Verfahren hat den Nachteil, daß Hydroxybutyrat in geringer Ausbeute zu L-Isoleucin umgewandelt wird. Es ist aber nicht bekannt, inwieweit das Hydroxybutyrat als Vorstufe diente und ob auch die D-Komponente des Racemats von Hydroxybutyrat verwertet wird.

Bei Einsatz von D,L-α-Hydroxybutyrat als Vorstufe erzielt man mit Corynebacterium glutamicum ATCC21193 eine Ausbeute von deutlich unter 70%, wenn man berücksichtigt, daß dieser Stamm auch ohne den Zusatz der Vorstufe L-Isoleucin produziert.

Dessen maximale Konzentration belief sich auf 7,8 g/l (DE-OS 1 910 426).

Im Vergleich dazu noch geringere Ausbeuten sind mit den in der DE-OS 1 912 819(= US-PS 3,671,396) beschriebenen Mikroorganismen verschiedener Genera wie z. B. Corynebacterium, Brevibacterium, Arthrobacter etc. zu erzielen.

Die geringen Ausbeuten kommen u. a. auch dadurch zustande, daß die Mikroorganismen das D-α-Hydroxybutyrat nicht verwerten.

Aus der DE-PS 1 543 877 ist ein Verfahren bekannt, bei dem man mit Brevibacterium ammoniagenes nach 5 Tagen aus einer 2 % DL-α-Hxdroxybuttersäure enthaltenden Fermentationsbrühe 8, 12 g reines L-Isoleucin erhält.

Aufgabe der Erfindung ist ein Verfahren zur fermentativen Herstellung von Isoleucin aus D, L-α-Hydroxybutyrat, bei dem aus dem Racemat L-Isoleucin in hoher Konzentration unter gleichzeitig guten Ausbeuten erreicht werden kann.

Gegenstand der Erfindung ist ein Verfahren zur Herstellung von L-Isoleucin aus D,L-α-Hydroxybutyrat durch Züchten eines Mikroorganismus vom Genus Corynebacterium glutamicum unter aeroben Bedingungen in einem wässrigen Kulturmedium, das assimilierbare Quellen für Kohlenstoff und Stickstoff und anorganische Salze enthält, und Isolieren des im Medium angereicherten L-Isoleucins, das dadurch gekennzeichnet ist, daß man einen Stamm verwendet, der auf einem festen Wachstumsmedium mit einem Gehalt von 0,1 bis 40 mg/ml D-Lactat kultiviert wurde, eine D-Lactatdehydrogenase-Aktivität von >0,1 bis 1,5 U/mg besitzt, und bei dem die Menge von 100 bis 250 μg/ml DL- oder L-Leucin in der Fermentationsbrühe limitierend wirkt.

Dies geschieht beispielsweise bei 30 °C für 3 bis 10 Tage auf Agarplatten der folgenden Zusammensetzung:

10 g/l Ammoniumsulfat, 5 g/l Harnstoff, 1 g/l Kaliumhydrogenphosphat, 0,25 g/l Magnesiumsulfat, 0,01 g/l Eisensulfat, 0,01 g/l Kalziumchlorid, 0,0001 g/l Biotin, 10 g/l D-Lactat, 15 g/l Agar-Agar, pH 7.

Vorteilhaft wählt man zur Kultivierung Mutanten von bekannten Corynebacteriumstämmen, die man durch UV-Bestrahlung oder durch Behandlung mit Mutagenen wie z. B. Ethylmethansulfonat, N-Methyl-N'-nitro-N-nitrosoguanidin erhält.

Insbesondere geeignet sind Mutanten von Corynebacterium glutamicum ATCC 13032 bzw. ATCC 14310, die durch die bekannte Behandlung der letztgenannten Stämme mit N-Methyl-N'-nitro-N-nitrosoguanidin erhalten wurden und bei der Deutschen Stammsammlung für Mikroorganismen in Göttingen unter

EP 0 248 238 B1

den Nummern DSM 3717 bzw. 3718 hinterlegt sind. Sie wachsen auf dem D-Lactathaltigen Nährmeidum und enthalten eine D-Lactatdehydrogenase-Aktivität von ca. 0,25 bzw. 0,4 U/mg, während die Ausgangsstämme nur ca. 0,1 U/mg enthalten.

(Die Aktivität wurde bestimmt gemäß Microbiological Reviews 44 (1980) 106 bis 139).

Geeignet für das erfindungsgemäße Verfahren sind Mutanten mit einer D-Lactatdehydrogenase Aktivität von >0,1 bis 1,5 U/mg.

Die Fermentation der isolierten Mutanten von Corynebacterium glutamicum führt man in Schüttelkulturen oder in Fermentern unter aeroben Bedingungen bei einer Reaktionstemperatur von 20 - 45° C und einem pH-Wert von 5 - 9 durch. Kalziumcarbonate und Ammoniumsalze sind für die Korrektur des pH's geeignet. Das Fermentationsmedium enthält eine Kohlenstoffquelle, D, L-$\alpha$-Hydroxybuttersäure, eine Stickstoffquelle und andere Elemente. Geeignete Kohlenstoffquellen für die Fermentation sind Zucker, Proteinhydrolysate und Proteine. D, L-$\alpha$-Hydroxybutyrat kann als freie Säure oder als Salz (wie z.B. als Natriumoder Kalziumsalz) in einer Konzentration von 0.01-50 g/l zum Reaktionsansatz dazugegeben werden.

Vorzugsweise geht man so vor, daß die Konzentration der Ausgangsverbindung 20 bis 40 g/l nicht übersteigt.

Um trotzdem eine bessere L-Isoleucin-Anreicherung in der Fermentationsbrühe zu erreichen, setzt man das D, L-$\alpha$-Hydroxybutyrat portionsweise unter Berücksichtigung der oben genannten bevorzugten Maximalwerte zu,bis das Fermentationsmedium 15 bis 30 g/l L-Isoleucin enthält.

Beispiele für geeignete Stickstoffquellen sind Harnstoffe, Ammoniumsalze organischer Säuren (z.B. Ammoniumacetat) oder Ammoniumsalze anorganischer Säuren (z.B. Ammoniumsulfate oder Ammoniumitrate).

Die Menge der Kohlenstoff- und Stickstoffquellen können von 0.001-40 g/l variieren. Auch können organische Nährstoffe (wie corn steep liquor, Pepton, Hefeextrakt) und/oder anorganische Bestandteile (wie Kaliumphosphat, Magnesiumsulfat, Vitamine wie Biotin, und Aminosäuren wie Leucin oder Valin) zur Nährlösung dazugegeben werden. Die Fermentation wird für 24 bis 192 Stunden durchgeführt, wobei L-Isoleucin in der Nährlösung akkumuliert.

In einer bevorzugten Ausführungsform setzt man dem Fermentationsmedium D,L oder L-Leucin zu und zwar in Mengen von 100 bis 250 $\mu$g/ml, insbesondere 200 $\mu$g/ml. Dadurch wird die Ausbeute an L-Isoleucin deutlich verbessert.

Das Leucin bleibt erhalten, da es offensichtlich nur regulatorisch wirkt.

Nach Beendigung der Fermentation wird das L-Isoleucin mit bekannten Maßnahmen isoliert und gereinigt.

Beispiel 1:

In einen 500 ml Erlenmeyerkolben mit 2 Schikanen wurden 100 ml einer Nährlösung mit folgender Zusammensetzung gegeben:

| | | |
|---|---|---|
| 40 | g/l | Glucose x $H_2O$ |
| 18 | g/l | D,L-$\alpha$-Hydroxybuttersäure |
| 20 | g/l | $(NH_4)_2SO_4$ |
| 0.5 | g/l | $K_2HPO_4$ |
| 0.25 | g/l | $MgSO_4$ x $7H_2O$ |
| 0.01 | g/l | $FeSO_4$ x $7H_2O$ |
| 0.01 | g/l | $MnSO_4$ x $4H_2O$ |
| 0.4 | mg/l | Biotin |
| 20 | g/l | $CaCO_3$ |
| 200 | mg/L | L-Leucin |

Glucose und die neutralisierte D,L-$\alpha$-Hydroxybuttersäure wurden getrennt sterilisiert, $CaCO_3$ trocken sterilisiert (8 h bei 150 °C) und der Nährlösung steril zugegeben.

4

5 ml einer 16 Stunden alten Vorkultur von Corynebacterium glutamicum mit erhöhter D-Lactatdehydrogenase Aktivität (DSM 3718), gewachsen in 100 ml Komplexmedium (20 g/l Glucose, 10 g/l Pepton, 10 g/l Hefeextrakt, 2,5 g/l NaCl, pH 7.4) in einem 500 ml Erlenmeyerkolben mit 2 Schikanen bei 30 °C und 130 rpm dienten als Inokulum der Hauptkultur, die bei 30 ° C und 130 rpm inkubiert wurde.

Nach einer Inkubationszeit von 72 Stunden enthielt das Fermentationsmedium 14.3 g/l L-Isoleucin. Das Fermentationsmedium des Kontrollansatzes ohne D,L-α-Hydroxybuttersäure enthielt kein L-Isoleucin.

Beispiel 2

Es wurde,wie in Beispiel 1 beschrieben, verfahren, jedoch enthielt die Nährlösung 24 g/l D,L-α-Hydroxybuttersäure. Nach einer Kultivierungsdauer von 72 Stunden enthielt das Fermentationsmedium 16.1 g/l L-Isoleucin.

Beispiel 3

Es wurde,wie in Beispiel 1 beschrieben, verfahren, jedoch enthielt die Nährlösung 400 mg/l L-Leucin. Nach der Fermentation konnten nur 6 g/l L-Isoleucin gefunden werden.

**Patentansprüche**

1.  Verfahren zur Herstellung von L-Isoleucin aus D,L-α-Hydroxybutyrat durch Züchten eines Mikroorganismus vom Genus Corynebacterium glutamicum unter aeroben Bedingungen in einem wässrigen Kulturmedium, das assimilierbare Quellen für Kohlenstoff und Stickstoff und anorganische Salze enthält, und Isolieren des im Medium angereicherten L-Isoleucins,
    dadurch gekennzeichnet, daß man einen Stamm verwendet, der auf einem festen Wachstumsmedium mit einem Gehalt von 0,1 bis 40 mg/ml D-Lactat kultiviert wurde, eine D-Lactatdehydrogenase-Aktivität von >0,1 bis 1,5 U/mg besitzt, und bei dem die Menge von 100 bis 250 $\mu$g/ml DL- oder L-Leucin in der Fermentationsbrühe limitierend wirkt.

2.  Verfahren gemäß Anspruch 1,
    dadurch gekennzeichnet, daß die Konzentration des D,L-α-Hydroxybutyrats 20 bis 40 g/l nicht übersteigt.

3.  Verfahren gemäß den Ansprüchen 1 und 2,
    dadurch gekennzeichnet, daß man das Corynebacterium glutamicum DSM 3717 oder DSM 3718 einsetzt.

**Claims**

1.  A process for the preparation of L-isoleucine from D,L-α-hydroxybutyrate by cultivation of a microorganism of the genus Corynebacterium glutamicum under aerobic conditions in an aqueous culture medium containing assimilable sources of carbon and nitrogen and inorganic salts, and isolation of the L-isoleucine accumulated in the medium, characterised in that the strain used is one which was cultivated on a solid growth medium containing from 0.1 to 40 mg/ml of D-lactate and has a D-lactate dehydrogenase activity of from >0.1 to 1.5 U/mg and in which the quantity of from 100 to 250 $\mu$g/ml of DL- or L-leucine in the fermentation broth has a limiting effect.

2.  A process according to Claim 1, characterised in that the concentration of the D,L-α-hydroxybutyrate does not exceed 20 to 40 g/l.

3.  A process according to Claims 1 and 2, characterised in that Corynebacterium glutamicum DSM 3717 or DSM 3718 is used.

**Revendications**

1.  Procédé de production d'isoleucine -L à partir d'hydroxybutyrate -D, L - α en cultivant un microorganisme du genre corynebactérium glutamicum sous des conditions aérobies dans un milieu de culture aqueuse, qui contient des sources assimilables de carbone et d'azote et des sels organiques, et en isolant l'isoleucine -L enrichie dans le milieu, caractérisé en ce que

- on utilise une souche qui a été cultivée sur un milieu de croissance solide avec une teneur de 0,1 à 40 mg/L de lactate -D, qui possède une activité de déshydrogénase de lactate -D supérieure à 0,1 et allant jusqu'à 1,5 U:mg et sur laquelle la quantité de 100 à 250 $\mu$g/ml de leucine -DL ou -L dans le bouillon de fermentation agit de façon limitée.

2. Procédé selon la revendication 1, caractérisé en ce que la concentration de l'hydroxybutyrate -D, 1 - $\alpha$ ne dépasse pas 20 à 40 g/l.

3. Procédé selon les revendications 1 et 2, caractérisé en ce qu'on utilise le corynebactérium glutamicum DSM 3717 ou DSM 3718.

6